# EUROPEAN PATENT APPLICATION

(11) **EP 3 936 054 A1**
(43) Date of publication of application: **12.01.2022**
(21) Application number: 20305784.9
(22) Date of filing: 08.07.2020
(51) Int. Cl.: A61B 8/08, A61K 38/17, A61K 38/30, A61P 11/00, A61K 35/42

(54) **METHOD OF PROGNOSIS OF BRONCHOPULMONARY DYSPLASIA IN PREMATURE INFANTS**

(71) Applicant: Assistance Publique - Hôpitaux de Paris, 75004 Paris (FR)
(72) Inventor: DE LUCA, Daniele, 92140 Clamart (FR); LOI, Barbara, 92140 Clamart (FR); YOUSEF, Nadya, 92140 Clamart (FR)
(74) Representative: Flesselles, Bruno F.G.

(57) **Abstract**

The present invention relates to a method for prognosis the risk of bronchopulmonary dysplasia in premature infant by adjusting a score obtained from lung ultrasound with the gestational age of the infant.

## Description

The present invention relates to a method of diagnosis and prognosis of the occurrence of bronchopulmonary dysplasia (BPD) in premature infants, through an easy test using lung ultrasound combined with the age of the infant.

Bronchopulmonary dysplasia (BPD) is a form of chronic lung disease that affects newborns, most often premature infants who need oxygen therapy. In BPD the lungs and the airways (bronchi) are damaged, causing tissue destruction (dysplasia) in the tiny air sacs of the lung (alveoli).

This disease is essentially due to an arrest of alveolarization, induced by mechanical ventilation (respirator) and long-term use of oxygen, and thus is evolving over time until a diagnosis is eventually made, when the infant is 36 weeks old (gestational age or postmenstrual age).

The classic diagnosis of BPD is assigned when the following criteria are met:
- The infant received supplemental oxygen for at least 28 days.
- The infant shows clinical signs of abnormal respiratory function.
- The infant needs oxygen or more aggressive types of respiratory support at 36 weeks post-menstrual age.
- Other criteria can be used, in particular the percentage of the needed oxygen, that will allow classification of the BPD into mild, moderate or severe.

There is no specific cure for BPD, so that it is important to minimize further lung damage and to allow healing of the lungs of the infants.

Some drug therapies may be used, such as diuretics, bronchodilators, corticosteroids, or cardiac medications. The infants are also more susceptible to viral infections and are generally treated to reduce risk of respiratory tract infections especially by the respiratory syncytial virus (RSV).

BPD is classically diagnosed at 36 weeks post-menstrual age, although preterm infants may be affected by chronic pulmonary insufficiency of prematurity (CPIP) on a continuum overtime starting after the first days of life and may need respiratory support well before the 36-week time-point.

It is thus important to detect presence BPD at an early stage so as to provide appropriate care (drug therapies or physical treatments such as proper oxygenation) to reduce the development of the disease and avoid, as much as possible, occurrence of severe cases. It is also important as an early detection may be used to select patients candidate to new therapies that are presently under advanced investigation. It is also possible to treat the specific baby with Vitamin A or steroids, which are currently available drugs for reducing BPD.

Lung ultrasound is a bedside technique commonly used in adult critical care since decades. Although with a certain delay compared to adult medicine, the technique paved its way in neonatology, as well. In fact, lung ultrasound significantly helps to diagnose the different types of neonatal respiratory failure, predicts clinical worsening besides efficaciously guide surfactant replacement, and is integrated in algorithms for clinical decision making. Based on these data, international guidelines for the use of lung ultrasound in neonatal and pediatric critical care have been recently released.

The aforementioned literature was essentially dedicated to the use of lung ultrasound in an acute setting, that is, in neonates with critical respiratory failure or needing urgent respiratory care, while few data are available on lung ultrasound in preterm infants with or at risk to develop chronic conditions, such as bronchopulmonary dysplasia (BPD).

Several studies have demonstrated that semi-quantitative lung ultrasound scores (LUS) accurately describe lung aeration both in neonates and adults (De Luca D. J Ultrasound Med. 2020;39(6):1235-1239. doi:10.1002/jum.15195). Furthermore, lung ultrasound is a radiation-free, quick and simple technique that can be used serially. Thus, it seems logical to use LUS to monitor the evolution of CPIP and predict clinical outcomes in preterm neonates. To date a small preliminary study has investigated the use of LUS to predict BPD in preterm neonates showing promising results (Alonso-Ojombarrena et Lubián-López, Pediatr Pulmonol. 2019 Sep;54(9):1404-1409). However, the sample size was small and there were a low number of moderate-severe patients with BPD. Furthermore, the authors suggest developing multiple tests depending on the gestational age.

The inventors have herein demonstrated that LUS is useful to monitor neonates with CPIP and predict BPD occurrence, and have developed a test that presents good accuracy, by developing a new score for lung ultrasound and combining it with the age of the premature infant. Contrary to the suggestions of Alonso-Ojombarrena et Lubián-López (*op*. *cit.*) who suggested multiple scores, the inventors showed that a single test (a single score) is applicable whatever the gestational age of the infants.

The invention thus relates to a method for determining whether a premature infant is susceptible to have bronchopulmonary dysplasia, comprising:
a) Obtaining a score from a lung ultrasound image obtained from the infant, wherein the score correlates with the
b) Obtaining the gestational age of the infant
c) Obtaining an end value by dividing the score of a) by the gestational age of b),
wherein the infant classified as at risk of having bronchopulmonary dysplasia when the end value is higher than a predetermined threshold (or cutoff) value.

The end value may also be described as final score in the present disclosure.

The above method is actually performed *ex vivo.* Consequently, in a preferred embodiment, the method doesn't include any step of harvest of data from the body of the infant, and used the ultrasound data (images) that have been previously gathered by an operator. This step on the human body is thus not comprised in (or excluded from) the method.

The method enables determining whether BPD will be present when assessed for when the infant reaches the age of 36 weeks (gestational age, or amenorrhea duration). The method can thus be qualified as being a method of prognosis of occurrence of BPD.

However, due to the fact that BPD is diagnosed after slow degradation of the state of the infant's lung, it can also be said that the method actually is a diagnosis method for CPIP, as it makes it possible to detect a lung condition in the infant that is already present when the method is performed.

In a first embodiment, the method is performed for an infant who is 7 days old. In another embodiment, the infant is 14 days old. In these two embodiments, the age is calculated after the birth of the infant.

As indicated above, the method combines a score obtained from lung ultrasound imagery with the gestational age of the infant.

Images from lung ultrasound are graded (from 0 to 3) and the grades are then summed up so as to obtain the score that is then divided by the gestational age of the infant.

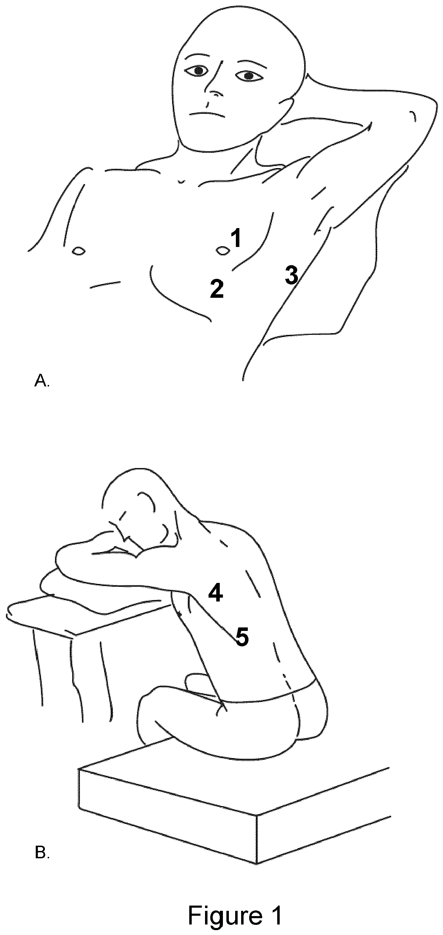

It is reminded that, when performing lung ultrasound, each hemithorax is divided into three regions (upper-anterior, lower-anterior and lateral) (Figure 1, see also Brat et al, JAMA Pediatr. 2015 Aug;169(8):e151797). Moreover, to be more accurate, posterior lung zones (upper-posterior and lower-posterior) may also be scanned. These were not examined in the classical score in order to be quicker and for the sake of simplicity, but they can be scanned by partially and gently tilting the baby on a side so gaining access to posterior chest. The extended lung ultrasound score will so have 10 areas of examination (5 areas for each lung (right and left)).

Ultrasound examination is performed for each area, and a grade from 0 to 3 is provided for each image.

Each grade is given as follows:
0 in the presence of only A-lines in the lung area (A-pattern)
1 in the presence of at least 3 well-spaced B-lines (B-pattern)
2 in the presence of crowded and coalescent B lines with or without consolidations limited to the subpleural space (severe B pattern)
3 when the ultrasound image shows a lung with extended consolidations.

A grade for each of the area of examination is thus obtained, and all grades are summed up in order to obtain a score (Lung Ultrasound Score). It is to be noted that this score could be divided by the number of examined areas or submitted to any other mathematical operation, without changing the information provided, that relates to the lung condition according to the multiple ultrasound view of it.

The person skilled in the art is aware of the way to examine lung ultrasound images, as the technique is known in the art. In particular, one of ordinary skill in the art is able to detect the A-lines and the B-lines (and the presence of lung consolidations).

A lines are a repetitive reverberation artifact of the pleura. They are seen in normal lung tissue.

B lines are long wide bands of hyperechoic artifact that originate at the pleural line and traverse the entire ultrasound screen vertically to the bottom of the screen. They erase A lines. As indicated above, there must be a minimum three B lines per view for their presence to be considered relevant.

A pulmonary consolidation is a region of normally compressible lung tissue that has filled with liquid instead of air. Compared to normal lung, consolidation on ultrasound has a relatively hypoechoic, heterogeneous echotexture with irregular borders and has a size of at least 0.5 cm.

As indicated, the person skilled in the art knows these patterns and is able to recognize them in lung ultrasound images. Multiple documents provide explanations and images of such images (Todd et al, Emergency Medicine. 2015 January;47(1):35-36; Francisco Neto et al, einstein. 2016;14(3):443-8, doi: 10.1590/S1679-45082016MD3557; Miller, BJA Education, 16 (2): 39-45 (2016); Piette et al, Curr Opin Anesthesiol2013, 26:20-30; Gargani and Volpicelli, Cardiovascular Ultrasound 2014,12:25; Havelock et al, Pleural procedures and thoracic ultrasound: British Thoracic Society pleural disease guideline 2010 Thorax 2010;65:i61-i76).

In a first embodiment, the score is obtained from the images obtained from each of the 3 areas (upper anterior, lower anterior, and lateral areas) of each lung of the infant (6 areas are thus studied).

A grade is given from 0 to 3, wherein 0 indicates A-pattern, corresponding to the presence of the only A-lines in the lung area, 1 corresponds to B-pattern, defined as the presence of at least 3 well-spaced B-lines), 2 corresponds to a severe B pattern, defined as the presence of crowded and coalescent B lines with or without consolidations limited to the subpleural space, and 3 corresponds to a lung with extended consolidations. The score, obtained from the sum of all obtained grades, ranges from 0 to 18.

In another embodiment, two other areas (upper posterior and lower posterior) are examined and the images thereof are graded as indicated above. In this embodiment, the score is thus obtained by grading, from 0 to 3, each of the 5 following areas (upper anterior, lower anterior, upper posterior and lower posterior and lateral areas) of each lung of the infant, and summing all obtained grades so as to obtain a total score ranging from 0 to 30.

The total score is then divided by the gestational age of the infant at the birth, i.e. the number of weeks of amenorrhea of the infant's mother so as to obtain a final score. The final score is then compared to a threshold (or cutoff value). When the final score is higher than the cutoff value, it is possible to conclude on the presence of the disease and it will be diagnosed when the infant reaches 36 weeks of gestational age. If the final score is lower than the cutoff value, the disease is not present and will not be diagnosed when the infant reaches 36 weeks of gestational age. Since BPD is an evolving disease, it is of interest to repeat the method when the infant is 7 days old and 14 days old. This makes it possible to detect the disease later (at 14 days) if not seen at 7 days, or alternatively to rule out presence of the disease if the method is positive at 7 days and negative at 14 days.

The quality of a test is generally determined by drawing a Receiving Operating Characteristic (ROC) curve and measuring the Area Under Receiving Operating Characteristic curve (AUROC). The ROC curve is drawn by plotting the sensitivity versus (1-specificity), after classification of the patients, according to the result obtained for the test, for different thresholds (from 0 to 1).

It is usually acknowledged that a ROC curve, the area under which has a value superior to 0.7, is a good predictive curve. The ROC curve has to be acknowledged as a curve allowing prediction of the quality of a test. It is best for the AUROC to be as closed as 1 as possible, this value describing a test which is 100 % specific and sensitive.

It is reminded that
(1) sensitivity is the probability that the diagnosis is positive in individuals having the phenotype sought (detection of true positives): the test is positive if the patient is having the phenotype. The sensitivity is low when the number of false negatives is high.
(2) specificity is the probability that the diagnosis is negative in the individuals not having the phenotype sought (non-detection of true negatives): the test is negative if the patient is not suffering from the disease or doesn't have the condition. The specificity is low when the number of false positives is high.
(3) Positive predictive value (PPV) is the probability of having the disease if the diagnostic test is positive (i.e. that the patient is not a false positive): the patient is having the phenotype when the test is positive.
(4) Negative predictive value (NPV): is the probability of not having the disease if the diagnostic test is negative (that the patient is not a false negative): the patient is not having the phenotype when the test is negative.

In order to obtain a good diagnostic test, it is important to both increase specificity and sensitivity. PPV and NPV depend on the prevalence of the phenotype in the population and of the specificity and sensitivity.

It is clear that the specificity, sensitivity, PPV and NPV depend on the threshold that is chosen. A "lower" threshold will decrease the specificity of the test (more false-positive), whereas a "higher" threshold will decrease the sensitivity of the test (some true positive will not be detected).

It is also to be noted that the method herein disclosed is not "gold-standard" tests, in the sense that the output (final score obtained as disclosed above) isn't a definitive answer as to the presence of BPD in infants. In fact, indicating that the method "predicts" the BPD at the gestational age of 36 weeks is similar to indicating that the probability of BPD for the infant is higher than 50% (i.e. that it is more likely that there will be BPD than not), or that the infant has a higher relative risk to have PBD than an infant with a score below the threshold. In fact, in view of the fact that the method is not gold-standard but is using indirect markers and is based on the statistics of the population studied for its development, some infants will be false-positive in the test, and some patients may be false-negative. This is the reason why the specific experience of the physician in interpreting the result is of importance for making the prognosis and deciding which kind of follow up and treatment is to be made to be made for each infant.

Thus, depending on the specificity, sensitivity, positive predictive value and negative predictive value of the tests, for various thresholds, the physician must interpret the result from the clinical and general context to be able to reach a conclusion. These tests are of great interest in providing a help to the physician when providing care to premature infants.

As an illustration, the examples provide some specific threshold for each of the situations described in details herein. As indicated, these values have been determined by the inventors as being appropriate (as having a good specificity and sensitivity), but other values can be used in different contexts (should the physician want to increase the sensitivity so as to avoid false negative, or the specificity to avoid false positive, when available treatments can have severe side effects).

This cutoff values can be used as indicated, or one can use the indicated values ±10% more preferable ±5%.

**Table 1. Appropriate threshold depending on age and investigated areas**

| **Age** | **Number of areas investigated** | **Cutoff value** |
|---|---|---|
| 7 days | 2x3 | 0.23 |
| | 2x5 | 0.43 |
| 14 days | 2x3 | 0.31 |
| | 2x5 | 0.59 |

The method is of particular interest for providing individualized care to the infants. Indeed, knowing that a specific baby is going to have BPD allows personalizing the respiratory support to reduce this risk.

It is also possible to treat the specific baby with Vitamin A or steroids, which are currently available drugs for reducing BPD.

Some new drugs are currently under development, and may be used in the babies that are identified as going to have BPD, either as treatment or in clinical trials. One can cite SP-D (surfactant protein D), intratracheal steroids (such as corticoids, in particular budesonide), or IGF-1 (insulin-like growth factor-1), alone or in combination.

4) knowning that that baby is going to have BPD allows to discuss with parents, inform them and organize the follow up of the baby both in hospital and after discharge

The invention thus relates to a method of treatment of a premature infant, comprising providing appropriate care (as indicated above) to the infant when the end value of the method as disclosed herein is above a predetermined cutoff.

The invention also relates to Vitamin A for use thereof for treatment or prevention of bronchopulmonary dysplasia in an infant for which the end value obtained from the method herein disclosed is above a predetermined cutoff value.

The invention also relates to steroids (especially when administered intratracheally) for use thereof for treatment or prevention of bronchopulmonary dysplasia in an infant for which the end value obtained from the method herein disclosed is above a predetermined cutoff value.

The invention also relates to SP-D for use thereof for treatment or prevention of bronchopulmonary dysplasia in an infant for which the end value obtained from the method herein disclosed is above a predetermined cutoff value. SP-D may be used alone of with steroids, in particular which are administered *via* intratracheal instillation (Zhong et al, Curr Med Sci, 2019 Jun;39(3):493-499. doi: 10.1007/s11596-019-2064-9; Bancalari et al, Am J Respir Crit Care Med, 2016 Jan 1;193(1):12-3. doi: 10.1164/rccm.201509-1830ED).

The invention also relates to IGF-1 for use thereof for treatment or prevention of bronchopulmonary dysplasia in an infant for which the end value obtained from the method herein disclosed is above a predetermined cutoff value.

### FIGURES

Figure 1: representation of the area for lung ultrasound examination. A. 1. upper anterior; 2. lower anterior; 3. Lateral; B. 4. upper posterior; 5. lower posterior.

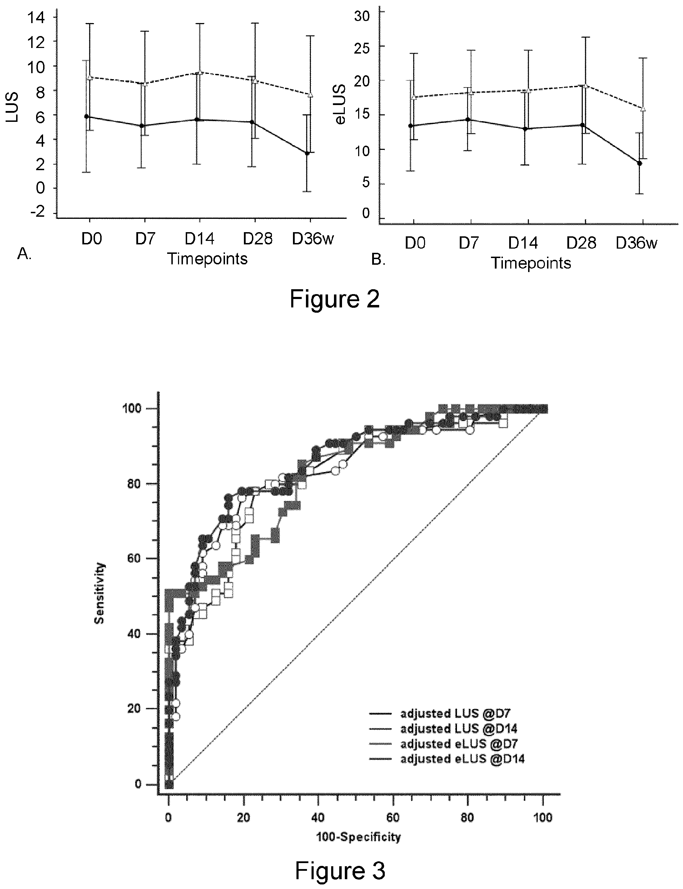

Figure 2: Lung ultrasound scores overtime. Panel A and B represent LUS and eLUS, respectively. Black and hatched lines represent BPD and control cohorts, respectively. Full circles and empty triangles represent means, while T-bars represent standard deviations. Symbols represent post-hoc between subjects' comparisons: * † ‡ # § p<0.001 between BPD and control cohort.
Figure 3: Receiver Operator Characteristics (ROC) curves for early (D7) and late (D14) prediction of BPD using lung ultrasound scores adjusted for gestational age. Different ROC curves are represented for adjusted LUS and eLUS calculated as lung ultrasound score-to-gestational age ratio, at seventh and fourteenth days of life. Hatched grey line represents the reference line. Area under the curves are similar (LUS @7 vs LUS @D14 p=0.855; LUS @D7 vs eLUS@D7 p=0.973; LUS @D7 vs eLUS@D14 p=0.462; LUS @D14 vs eLUS @D7 p=0.883; LUS @D14 vs eLUS @D14 p=0.145; eLUS @D7 vs eLUS @D14 p=0.403). Open squares: LUS @D7; open circles: LUS @D14; plain squares: eLUS @D7; plain circles: eLUS@D14.
   Abbreviations: BPD: bronchopulmonary dysplasia; D7: seventh day of life; D14: fourteenth days of life; eLUS: extended lung ultrasound score; LUS: lung ultrasound score.
Figure 4: Reliability data for gestational age-adjusted lung ultrasound scores for early (D7) and late (D14) prediction of BPD. Cut-off values associated with minimal false negative and positive results are shown. Data are expressed with 95% confidence interval (CI). Abbreviations: AUC: area under the ROC curve; BPD: bronchopulmonary dysplasia; D7: seventh day of life; D14: fourteenth days of life; eLUS: extended lung ultrasound score; LR: likelihood ratio; LUS: lung ultrasound score; PV: predictive value.

### EXAMPLES

### MATERIAL AND METHODS

### Study design

A multicenter, pragmatic, international, observational, non-invasive, prospective, diagnostic accuracy study was conducted in five academic tertiary referral neonatal intensive care units (NICU) in France and Italy. The NICU at Paris Saclay University Hospital served as coordinating center. The study was approved by local ethical boards and parental/guardian consent was obtained following local regulations. The study was registered in the ISRCTN Registry and details are available there. The study was pragmatic as the participation did not change the clinical management, which was provided according to local NICU protocols, essentially based on optimal prenatal care and international guidelines for neonatal resuscitation and respiratory management of preterm neonates. Participating NICU teams are proficient in lung ultrasound and routinely use the technique in their clinical care, according to clinicians' evaluation.

### Patients

All extremely preterm inborn neonates with gestational age ≤30 weeks whose parents agreed to participate were considered eligible for the study, if they do not have any of the following *a priori* exclusion criteria: 1) complex congenital malformations; 2) chromosomal abnormalities; 3) pulmonary hypoplasia; 4) congenital anomalies of surfactant proteins or any other suspected congenital lung disorders. At the end of recruitment, all data were sent to the coordinating centre, merged and reviewed to check for completeness and accuracy. Local investigators were contacted if clarification or more data were needed and infants were excluded *post hoc* in case of: 1) death or transfer to other hospitals before 36 weeks post-menstrual age; 2) missing data needed for the BPD diagnosis.

### Data collection

Data were prospectively collected into customized, secured, electronic spreadsheets by local investigators in each center. Data were completely anonymous in accordance with local and European privacy regulations, with local investigators maintaining an identification log. Basic demographics and common clinical data obtained during routine care were collected. Lung ultrasound, ventilatory and gas exchange data at definite timepoints were also registered (see below). A detailed list of collected data and standardised definitions used in the study is available in the ISRCTN Registry.

### Lung ultrasound protocol

Lung ultrasound was performed upon NICU admission (day 0 (DO)) and at seven (D7), fourteen (D14) and twenty-eight (D28) days of postnatal age. D0 lung ultrasound was performed upon NICU admission and always before surfactant administration, if any. A final ultrasound was performed at 36 weeks post-menstrual age (36W). Lung ultrasound was performed in some centers with "hockey stick" micro-linear (15 MHz) and in others with a broadband linear (10 MHz) probe, according to the availability. At each time-point, a LUS specifically created and validated for newborn infants was calculated in real-time: this score is based on classical lung ultrasound semiology and is calculated over 6 chest areas (3 per each side, ranging from 0 to 18), as we previously described (Brat et al, *op*. *cit*). Additionally, investigators using a micro-linear probe also calculated an extended score (eLUS) over 10 chest areas (5 per each side, ranging from 0 to 30) including the scan of the upper posterior and lower posterior chest areas. Lung ultrasound patterns used for score calculation are described in the study definitions and illustrative examples are provided in Brat et al (*op. cit.*)*.* Scans were performed by automatically adjusting the gain; depth and focus were set according to patients' size and the sign of interest. Lung ultrasound was performed in incubators, when the neonate was quiet and lying supine or slightly tilted to scan the posterior zones, during routine clinical care to minimize discomfort. Within 1 hour from lung ultrasound, if the patient had normal temperature and peripheral perfusion, blood gases (transcutaneous partial pressure of oxygen [PtcO₂] and carbon dioxide [PtcCO₂]) were measured with adequately calibrated transcutaneous devices (TCM4^{®}, Radiometer Medical, Copenhagen, Denmark), used according to the American Association for Respiratory Care guidelines and manufacturer's recommendations. Probes were applied until the achievement of a stable measurement and anyway for a maximum of 15 minutes, and, at the same time, mean airway pressure (P_{aw}) and vital parameters were recorded. During transcutaneous measurements, ventilatory parameters were not changed and, for neonates receiving non-invasive respiratory support, pressure leaks have been minimized, by using appropriately sized interfaces and closing the mouth with gentle pressure on the jaw.

### Outcomes

The primary outcomes were: (1) to efficaciously monitor lung aeration in neonates with CPIP by describing the relationship between lung ultrasound scores and gas exchange at different time points; 2) to demonstrate accuracy of LUS to predict BPD at 36 weeks post-menstrual age. Secondary outcome was to compare the performance of the classical and extended LUS to monitor lung aeration and predict BPD. BPD was diagnosed according to Jobe and Bancalari's criteria (Jobe and Bancalari. Am J Respir Crit Care Med. 2001;163(7):1723-1729) by a clinician blinded to LUS data.

### Calculations and statistics interim analysis

The following indices were calculated to describe oxygenation (Brat et al (*op*. *cit*.)): (1) PtcO₂ to FiO₂ (P/F) ratio; (2) Alveolar-arterial gradient = PA - PtcO₂, where PA indicates alveolar oxygen pressure and is given by (FiO₂ × [760 - 47]) - (PtcCO₂/0.8); (3) arterial to Alveolar (a/A) ratio = PtcO₂/PA; and (4) oxygenation index (OI) = P_{aw} × FiO₂ × 100/PtcO₂.

Sample size was calculated for the two primary outcomes as follows. To monitor lung aeration and function we targeted a correlation coefficient between LUS and Ol of at least 0.6, based on previous data obtained in a similar population of extremely preterm neonates (De Martino et al. Pediatrics September 2018, 142 (3) e20180463). To predict BPD occurrence, we targeted an area under curve (AUC) of at least 0.7, and considering as null hypothesis the prediction by chance (AUC=0.5) and a positive/negative (i.e.: BPD/no BPD) case ratio of 1. For both calculations α and β were set at 0.05 and sample size resulted 98 and 100 for the two outcomes respectively. Given the easiness to recruit and the time needed to diagnose BPD we enlarged the recruitment to ensure having an equal number of positive and negative cases. An *interim* analysis was performed at 50% of the enrolment and no changes to the study protocol were made.

Data were expressed with mean (standard deviation) or median [interquartile range], as appropriate. Basic population data were compared with χ² or Fisher and Student or Mann-Whitney test, as appropriate. Lung ultrasound scores calculated at the various timepoints were compared with repeated measures-ANOVA, using the BPD diagnosis as between subjects' factor and followed by Bonferroni *post hoc* test. Correlation analyses with lung ultrasound scores were performed using Spearman coefficients, followed by multivariate linear regressions with backward-stepwise method, adjusting for gestational age and the diagnosis of BPD. Covariates were removed from the model if *p*-value was >0.10. Gestational age was chosen as covariate because of its association with BPD; birth weight was not included because it is correlated with gestational age and creates significant multicollinearity. Results will be graphically shown in scatter plots with trendline generated by local regression smoothing procedure (Epanechnikov's kernel with 85% span).

Receiver operator characteristics (ROC) procedure analyzed the accuracy of lung ultrasound scores on different timepoints to predict BPD: curves were compared with DeLong's method and results are reported as area under the curve (AUC and 95% confidence interval). Then, lung ultrasound scores with highest AUC were entered in multivariate, logistic, backward-stepwise models together with gestational age and their interaction term. Covariate treatment was as above; goodness-of-fit was evaluated with Hosmer-Lemeshow test. Results were used to create gestational age-adjusted lung ultrasound scores. They were subjected to ROC analyses and post-test probability was estimated using the Fagan nomogram. Analyses were performed with SPSS 25.0 (SPSS Inc, Chicago, IL - USA), MedCalc 13.3 (MedCalc bvba, Ostend, Belgium), and GPower 3.1 (HHU, Dusseldorf, Germany). *p*-values<0.05 was considered significant.

### RESULTS

One-hundred and seventy-nine neonates were eligible and 32 were excluded (for death (7) or transfer (4) before 36 weeks post-menstrual age, lack of BPD data (21)), thus 147 neonates were finally included and analyzed. Table 2 shows the basic population data: infants with and without BPD have similar baseline characteristics, but BPD infants are more preterm. BPD was mild, moderate and severe in 24 (16.3%), 32 (21.8%) and 16 (10.9%) neonates, respectively. A subgroup of 115 neonates (57 in BPD and 58 in the control cohort, respectively) underwent both LUS and eLUS calculations. Basic patients' characteristics were similar between recruiting centers. Neonates were stable during lung ultrasound and data collection and no problem was noticed.

**Table 2. Basic population details. Data are expressed as mean (standard deviation) or median [interquartile range] or number (%). Abbreviations: BPD: bronchopulmonary dysplasia; NICU: neonatal intensive care unit; SGA: small for gestational age.**

| | **Whole population (147)** | **BPD cohort (72)** | **Control cohort (75)** | ***p*** |
|---|---|---|---|---|
| Gestational age (weeks) | 27.3 (1.9) | 26.3 (1.9) | 28.1 (1.5) | <0.001 |
| Birth weight (g) | 954 (289) | 812 (252) | 1089 (257) | <0.001 |
| SGA neonates | 37 (25%) | 22 (30.6%) | 15 (20%) | 0.140 |
| Male sex | 77 (52%) | 38 (52.8%) | 39 (52%) | 0.925 |
| Antenatal steroids | 123 (84%) | 61 (84.7%) | 62 (82.7%) | 0.736 |
| Cesarean section | 96 (65%) | 44 (61.1%) | 52 (69.3%) | 0.295 |

Both LUS and eLUS significantly vary between the timepoints (both overall *p*<0.0001, within subjects' contrast) and *post-hoc* tests show that they are lower at 36W than at D0 (*p*=0.003 for LUS; *p*=0.05 for eLUS), D7 (both *p*<0.001), D14 (both *p*<0.001) and D28 (both *p*<0.001). Diff33w-28 LUS and eLUS are also different between BPD and control cohorts (both overall *p*<0.0001, between subjects' contrast). Figure 2 shows the two lung ultrasound scores overtime and between subject *post-hoc* comparisons.

LUS significantly correlate with oxygenation metrics and with PtcCO₂ (except on D7) and this is confirmed upon adjustment for gestational age and BPD diagnosis. LUS is also correlated to Silverman's score at D0 (ρ=0.432, *p*<0.0001; β=0.18, *p*<0.0001), D7 (ρ=0.45, *p*<0.0001; β=0.1, *p*=0.001), D14 (ρ=0.394, *p*<0.0001; β=0.07, *p*=0.014), D28 (ρ=0.544, *p*<0.0001; β=0.13, *p*<0.0001) and 36W (ρ=0.59, *p*<0.0001; β=0.16, *p*<0.0001). Similar correlations were found using eLUS, except that for PtcCO₂, which did not correlate with eLUS at any time-point (not shown).

For the prediction of BPD, a preliminary analysis showed that AUC of LUS and eLUS were slightly higher for the scores calculated on the seventh and fourteenth day of life BPD. When these scores were adjusted for gestational age in multivariate logistic models a significant interaction between lung ultrasound scores and gestational age was evident, with increasing gestational age and lung ultrasound score being associated with reduced and augmented BPD occurrence, respectively.

Thus, gestational age-adjusted lung ultrasound scores were calculated as lung ultrasound score-to-gestational age ratio for early (D7) or late (D14) prediction of BPD. They resulted significantly associated to BPD, both at D7 (adjusted LUS: OR: 803 (95%CI: 55-11625), *p*<0.0001; adjusted eLUS: 861 (64-11586), *p*<0.0001) and at D14 (adjusted LUS: OR: 917 (95%CI: 69-12183), *p*<0.0001; adjusted eLUS: 815 (6704-9484), *p*<0.0001).

Figure 3 shows ROC curves of these adjusted scores: their AUCs are not significantly different from each other.

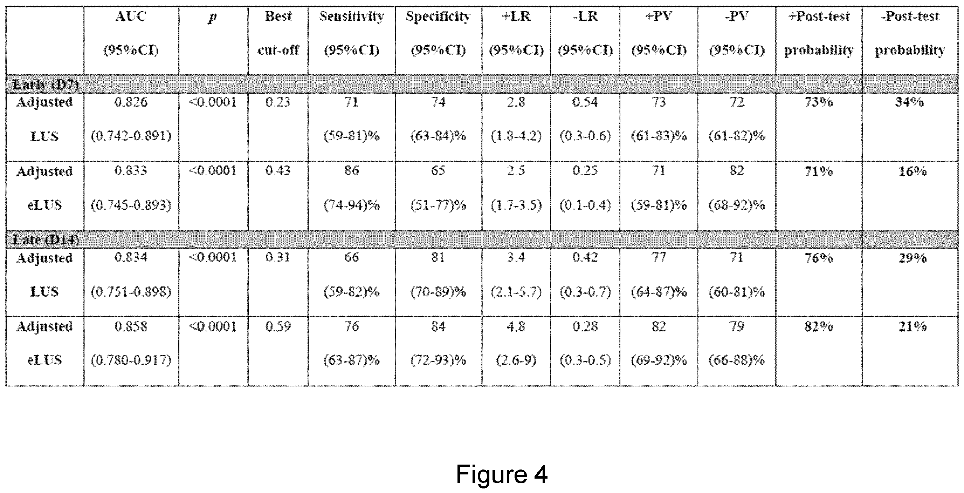

Figure 4 shows reliability data and best cut-off values for the adjusted scores.

## Claims

1. An *ex vivo* method for determining whether a premature infant is having or is susceptible to have bronchopulmonary dysplasia (BPD), comprising:
a) Obtaining a score from a lung ultrasound image obtained from the infant, wherein the score correlates with the
b) Obtaining the gestational age of the infant
c) Obtaining an end value by dividing the score of a) by the gestational age of b),
wherein the infant classified as at risk of having bronchopulmonary dysplasia when the end value is higher than a predetermined threshold [cutoff] value.

2. The method of claim 1, wherein the infant is 7 days old.

3. The method of claim 1, wherein the infant is 14 days old.

4. The method of any one of claims 1 to 3, the score of a) is obtained by providing a grade from 0 to 3 to each of the 3 areas (upper anterior, lower anterior, and lateral areas) of each lung of the infant, wherein 0 indicates A-pattern, corresponding to the presence of the only A-lines in the lung area, 1 corresponds to B-pattern, defined as the presence of at least 3 well-spaced B-lines), 2 corresponds to a severe B pattern, defined as the presence of crowded and coalescent B lines with or without consolidations limited to the subpleural space, and 3 corresponds to a lung with extended consolidations, and summing all obtained grades so as to obtain a total score ranging from 0 to 18.

5. The method of any one of claims 1 to 3, the score of a) is obtained by providing a grade from 0 to 3 to each of the 5 areas (upper anterior, lower anterior, upper posterior and lower posterior and lateral areas) of each lung of the infant, wherein 0 indicates A-pattern, corresponding to the presence of the only A-lines in the lung area, 1 corresponds to B-pattern, defined as the presence of at least 3 well-spaced B-lines), 2 corresponds to a severe B pattern, defined as the presence of crowded and coalescent B lines with or without consolidations limited to the subpleural space, and 3 corresponds to a lung with extended consolidations, and summing all obtained grades so as to obtain a total score ranging from 0 to 30.

6. The method of any one of claims 1, 2 and 4 in its dependency with claim 2, wherein threshold is 0.23.

7. The method of any one of claims 1, 2 and 5 in its dependency with claim 2, wherein threshold is 0.43.

8. The method of any one of claims 1, 3 and 4 in its dependency with claim 3, wherein threshold is 0.31.

9. The method of any one of claims 1, 3 and 5 in its dependency with claim 3, wherein threshold is 0.59.

10. Vitamin A for use thereof for treatment or prevention of bronchopulmonary dysplasia in an infant for which the end value obtained from the method of any one of claims 1 to 5 is above a predetermined cutoff value.

11. Insulin-like Growth Factor-1 for use thereof for treatment or prevention of bronchopulmonary dysplasia in an infant for which the end value obtained from the method of any one of claims 1 to 5 is above a predetermined cutoff value.

12. Surfactant protein D for use thereof for treatment or prevention of bronchopulmonary dysplasia in an infant for which the end value obtained from the method of any one of claims 1 to 5 is above a predetermined cutoff value.

13. Steroids, (in particular budesonide) for use thereof for treatment or prevention of bronchopulmonary dysplasia in an infant for which the end value obtained from the method of any one of claims 1 to 5 is above a predetermined cutoff value.

14. Steroids for use thereof according to claim 13, which are administered by intratracheal instillation.
